# EUROPEAN PATENT SPECIFICATION

(11) **EP 3 226 920 B1**
(45) Date of publication and mention of the grant of the patent: **25.03.2020**
(21) Application number: 15823775.0
(22) Date of filing: 04.12.2015
(51) Int. Cl.: A61L 9/12

(54) **AIR FRESHENER**
LUFTERFRISCHER
DISPOSITIF DE DÉSODORISATION

(30) Priority: 05.12.2014 IT MO20140355
(43) Date of publication of application: 11.10.2017
(73) Proprietor: Sarong Societa' Per Azioni, 42046 Reggiolo (IT)
(72) Inventor: BARTOLI, Andrea, 42123 Reggio Emilia (IT)
(74) Representative: Molinari, Marinella
(86) International application number: PCT/IB2015/059365
(87) International publication number: WO 2016/088095

(56) References cited:
- WO-A1-2013/076033
- WO-A1-2014/148999
- US-A- 4 161 283
- US-A- 4 534 509
- US-A- 5 126 070
- US-A- 5 242 111
- US-A1- 2007 055 216
- US-A1- 2011 180 621

## Description

The invention relates to an air freshener containing a volatile substance to be released into the air to freshen a room.

Different types of air freshener devices are known that enable a volatile substance to be diffused in an environment, such as an indoor environment, to freshen the air thereof. A first type of freshener devices are those that require a source of energy, for example electric or thermal energy. A second type are those that have to be activated by people. A further distinction of the latter is made between devices that have to be activated whenever it is desired to diffuse a scent in the air, like aerosol systems, and devices that require a single initial activation after which they continue to release a fragrance for a certain time, like monodose air fresheners for wardrobes or for motor vehicles.

Air freshener devices are known for diffusing a fragrance in the form of a volatile substance comprising a tank, to the edge of which a gas permeable sheet or membrane is welded that is protected by a barrier sheet that is impermeable to gases and is removable. One drawback of this type of device is that the removable sheet can get separated from the permeable membrane at undesired moments, for example during transport and storage of the containers, this causing the volatile substance to be consumed before the device is used by the consumer.

Another drawback is that when the barrier sheet is removed to activate the device a fragrance release peak occurs that is no longer obtainable in subsequent periods.

WO2010/120960 discloses an air freshener device of the continuous emission type that comprises a tank containing a volatile material enclosed by a breakable substrate made of aluminium/PET glued or welded to the tank. A rupture element comprising perforating elements is positioned near the breakable substrate and a breathable membrane encloses the tank, the breakable substrate and the rupture element. A user can drive the rupture element manually, by pressing the rupture element and thus breaking the breakable substrate; the perforating elements create openings in the breakable substrate through which the volatile material exits the tank and reaches the breathable membrane.

One drawback of this device is the relative structural and constructional complexity, the device consisting of a certain number of components, one of which is the rupture element; the latter, in fact, has to be produced before and separately from the tank and requires a dedicated convey and handling line in order to be inserted into the tank production line so that it can be included in the device, this making the production cost of the device relatively high.

A further drawback is that, once the breakable substrate has been broken, it is no longer possible to vary the intensity of the emission of the fragrance.

WO2013076033(A1) discloses a fragrance diffuser, particularly for small spaces, comprising a main body which forms an expansion seat and a containment chamber separated from the expansion seat, for containing a fluid, and a membrane which faces the external environment and is at least partially proximate to the expansion seat; at least one passage channel is interposed between the containment chamber and the expansion seat, provided internally with at least one barrier partition for the fluid. The fragrance diffuser has a closed configuration in which the partition is active to stop the passage of the fluid from the containment chamber to the expansion seat and a configuration for use in which the partition is inactive for the passage of the fluid from the containment chamber to the membrane, passing through the expansion seat.

One object of the invention is to produce an air freshener that enables known air freshener devices to be improved.

Another object is to obtain an air freshener for releasing a volatile substance that is relatively simple and has a low production cost.

Still another object is to produce an air freshener that enables a gradual release of the volatile substance to be obtained but also the intensity of the fragrance emitted even after the first activation to be increased.

According to the invention an air freshener is provided according to what is claimed in the enclosed claims.

Owing to the peelable closing film, it is not necessary to include inside the air freshener any element that triggers the breakage of the closing film itself, this enabling an air freshener device to be obtained that is simpler to produce, and at a cost that is lower than that of known air freshener devices.

Further, the user can press the shell several times on different occasions, increasing the detached section of the closing film and thus varying the quantity of volatile substance that passes from the cavity to the chamber; this enables the intensity of the fragrance released into the environment to be increased on subsequent occasions.

The shaped portion gives the peelable closing film a preferential detachment position, this ensuring that when the cavity is pressed the volatile substance enters the chamber gradually.

The invention can be better understood and implemented with reference to the attached drawings that illustrate an embodiment thereof by way of non-limiting example in which:

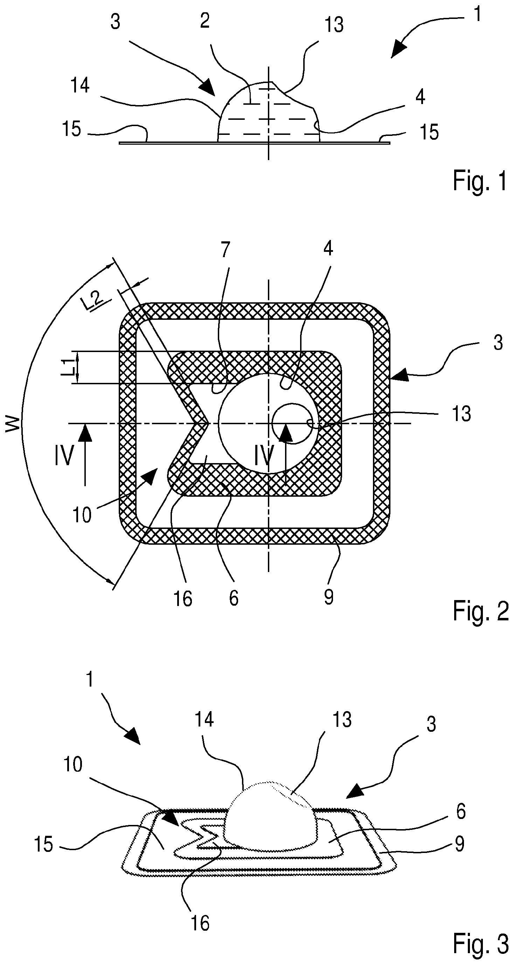

Figure 1 is a side view of an air freshener for freshening an environment;
Figure 2 is a top view of the air freshener of Figure 1
Figure 3 is a perspective view of the air freshener of Figure 1;
Figure 4 and Figure 5 are enlarged sections that are not to scale taken along the plane IV-IV of Figure 2, illustrating different configurations of the air freshener.

The Figures show an air freshener 1 containing a volatile substance 2 for diffusing a fragrance in an environment, such as, for example, a room, the interior of a motor vehicle, a wardrobe, a draw or any desired environment. The volatile substance is a fluid that can vaporise at ambient temperature, such as a perfume, an essential oil or a sublimable powder.

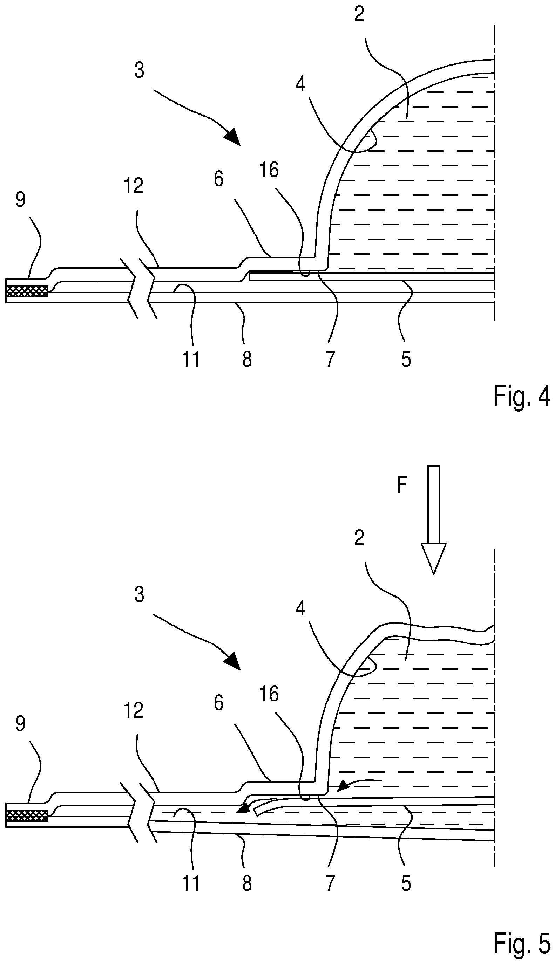

The air freshener 1 comprises a shell 3, which is impermeable to gas, defining a cavity 4 containing the volatile substance 2. The shell 3 has an outer surface having a spherical area 14 at the cavity 4 and a substantially flat area 15 that extends around the spherical area 14. With reference to Figures 4 and 5, a closing film 5 is attached to the shell 3 along a first boundary region 6 surrounding the cavity 4. The air freshener 1 further comprises a breathable film 8 attached to the shell 3 along a second boundary region 9 of the shell 3. The latter surrounds the first boundary region 6 so that the breathable film 8 is on the outside and the closing film 5 is arranged between the breathable film 8 and the shell 3. Considering the illustrated position of the air freshener 1, a chamber 11 is bounded below by the breathable film 8 and above by the closing film 5 and by a wall 12 of the shell 3, this wall 12 being interposed between the first boundary region 6 and the second boundary region 9. An opening 7 of the cavity 4 faces the chamber 11 and is closed by the closing film 5. By proceeding from the inside to the outside of the shell 3, the chamber 11 is thus more external and subsequent to the cavity 4.

The shell 3 is a thermoformed body obtained from a sheet material by thermoforming, for example from a multilayered sheet of PE/PET. The closing film 5 is impermeable to gas and can be a film of aluminium/PE. The breathable film 8 is for example of Teslin or other. The closing film 5 is attached in a peelable manner at the first boundary region 6. In particular, the closing film 5 is thermowelded along the first boundary region 6 according to known time and temperature parameters that enable the resulting weld to withstand a certain separating force but enable the closing film 5 to detach once the predefined separating force is exceeded. In the second boundary region 9 the breathable film 8 is attached permanently, for example by welding or adhesive.

Through the effect of a pressure increase inside the cavity 4 the closing film 5 detaches from the first boundary region 6 enabling the volatile substance 2 to exit the cavity 4 and go to the chamber 11.

In order to check a detachment position of the closing film 5 from the shell 3, the first boundary region 6 is provided with a shaped portion 10, which reduces the resistance to the detachment of the closing film 5 from the shell 3; in other words, by increasing the pressure inside the cavity 4, the closing film 5 detaches at the shaped portion 10 before other areas of the first boundary region 6. The shaped region 10 is "V"-shaped. The tip of the "V" of the shaped portion 10 points towards the centre of the cavity 4. Between a concave portion of the cavity 4, for example a substantially spherical portion, and the shaped portion 10 an interspace 16 is defined bounded by the shell 3 and by the closing film 5 in which the volatile substance 2 is present that acts directly against the shaped portion 10.

At the shaped portion 10, the first boundary region 6 has a transverse extent that is less than other portions of the first boundary region 6, i.e. it has a width, measured orthogonally to the perimeter edge of the opening 7, which is less. If for example, the width L1 of the first boundary region 6 can be comprised between 5 mm and 11 mm, at the shaped portion 10 the first boundary region has a width L2 of about 3 mm. The arms of the "V" form an acute angle W that can be about 120°.

The shaped portion 10 can also have a shape that is different from the shape illustrated, for example the shaped portion 10 can be "U"-shaped, or "W"-shaped.

The shaped portion 10 defines a preferential detachment area of the closing film 5 and enables the volatile substance 2 to flow gradually from the cavity 4 to the chamber 11, so that the air freshener 1 can emit the fragrance in a prolonged manner over time.

The air freshener 1 is activated manually by a user by applying a force F to the shell 3, as shown in Figure 5. An outer surface of the shell 3 has a depression 13 that is suitable for receiving the finger of the user.

By pressing shell 3, the pressure of the volatile substance in the cavity 4 consequently increases, which, after exceeding a predefined value, causes a first detachment of the peelable closing film 5 at the shaped portion 10. The volatile substance exits the cavity 4 through the area of opening 7 left free by the closing film 5 and sprinkles the chamber 11, wetting the breathable film 8, which, being permeable to gases, thus enables the fragrance to exit the air freshener 1 and spread around the external environment.

When the volatile substance 2 passes from the cavity 4 to the chamber 11, the shell 3 is deformed and the cavity 4 reduces in volume whereas the chamber 11, in which the volatile substance 2 expands, increases in volume, increasing the space between the opening 7 and the breathable film 8, a space that is occupied by the volatile substance 2. This occurs owing to the flexibility of the shell 3 and to the flexibility of the breathable film 8.

The chamber 11 can contain a material that is mixable with or reactant to the volatile substance 2, for example a material in powder or a liquid, possibly perfumed, to obtain a combination of perfumes. Alternatively, the chamber 11 can contain a porous material or an absorbent material that receives the volatile substance 2 or a material impregnated with a further substance that can mix with or react to the volatile substance 2 to activate the evaporation of a perfume.

The force F applied from the outside of the shell 3 that is necessary for activating the air freshener 1 moves only a certain quantity of volatile substance 2 into the chamber 11, whereas a great quantity of volatile substance 2 still remains contained in the cavity 4.

By pressing again on the outer surface of the shell 3, a further quantity of volatile substance can reach the breathable film 8. This operation is repeatable several times, the number of which depends on the force F that is applied each time to the shell 3. Compared with known air freshener devices, with the air freshener 1 it is thus possible to increase the intensity of the perfuming emitted even in periods that are subsequent to the activation of the air freshener 1.

Alternatively to the application of further pressure on the shell 3, in order to increase the intensity of the fragrance emitted once the air freshener 1 has been activated, the user can change the position of the air freshener 1 in space, for example by rotating the air freshener 1, so that the volatile substance 2 drops by gravity from the area of the opening 7 left free by the already detached closing film 7.

In one embodiment that is not shown, the air freshener 1 can comprise in a single thermoformed body or shell a plurality of protruberances corresponding to respective cavities, closed by respective peelable closing films, which face one another on the same chamber, the latter being closed by a single breathable film. In this embodiment, the user can press the protruberances on different occasions to prolong the deodorant effect when the perfume in an initially activated protruberance has become exhausted. In one embodiment the cavities contain different fragrances and the user can decide which protruberance to press and possibly, by pressing several protruberances, mix the contents of the protruberances in the same chamber to obtain a mixture of fragrances.

In order to produce the air freshener 1 a step of thermoforming the shell 3 has to be initially provided. The closing film 5 is obtained from a reel that is cut by a punch and the blank is then positioned on the thermoformed body of the shell 3 where it is welded to close the cavity 4. Subsequently, the breathable film 8 is attached, in particular welded, to the second boundary region 9 and cut to the preset shape and dimensions.

The production of the air freshener 1 is thus simpler than that of air freshener devices of known type that require an additional rupture element of the closing film.

## Claims

1. Air freshener comprising a thermoformed shell (3) defining a cavity (4) containing a volatile substance (2), a closing film (5) attached to said shell (3) in a first boundary region (6) surrounding said cavity (4) to close an opening (7) of said cavity (4), a breathable film (8) attached to said shell (3) in a second boundary region (9) surrounding said first boundary region (6) so that between said closing film (5), a wall (12) of said shell (3) and said breathable film (8) a chamber (11) is defined, said wall (12) being interposed between said first boundary region (6) and said second boundary region (9), said closing film (5) being peelable at said first boundary region (6) and said first boundary region (6) comprising a shaped portion (10) defining a preferential area of detachment of said closing film (5) from said shell (3) to enable said volatile substance (2) to pass from said cavity (4) to said chamber (11) as said air freshener (1) is activated by a force (F) pressing on said shell (3) **characterized in that** said chamber (11) is bounded on one side by said breathable film (8) and on the opposite side by said closing film (5) and by said wall (12) of said shell (3).

2. Air freshener (1) according to claim 1, wherein said shaped portion (10) has a shape like a "V" with a tip pointing towards the centre of said cavity (4).

3. Air freshener (1) according to claim 1, or 2, wherein said first boundary region (6) has a smaller width (L2) at said shaped portion (10).

4. Air freshener (1) according to any preceding claim, wherein an outer surface of said shell (3) comprises a depression (13) suitable for receiving the finger of a user.

5. Air freshener (1) according to any preceding claim, wherein said chamber (11) contains a material mixable with said volatile substance (2) or receiving said volatile substance (2) selected from a group comprising: a liquid, a powder, an absorbent material, a porous material, a material impregnated with a further substance.

6. Air freshener (1) according to any preceding claim, and further comprising a further cavity containing an additional volatile material, said further cavity being facing said chamber (11) and closed by a respective further closing film.

## Patentansprüche

1. Luftauffrischer mit einem thermogeformtem Gehäuse (3), das einen Hohlraum (4) definiert, der eine volatile Substanz (2) enthält, wobei eine an dem Gehäuse (3) in einem ersten Grenzbereich (6) befestigt ist, der den Hohlraum (4) umgibt, um eine Öffnung (7) des Hohlraumes (4) zu verschließen, wobei eine atmungsfähige Folie (8) an dem Gehäuse (3) in einem zweiten Grenzbereich (9) befestigt ist, der den ersten Grenzbereich (6) umgibt, so dass zwischen der Verschlussfolie (5), einer Wand (12) des Gehäuses (3) und der atmungsfähigen Folie (8) eine Kammer (11) definiert ist, wobei die Wand (12) zwischen dem ersten Grenzbereich (6) und dem zweiten Grenzbereich (9) eingeschlossen ist, wobei die Verschlussfolie (5) von dem ersten Grenzbereich (6) ablösbar ist und der erste Grenzbereich (6) einen geformten Bereich (10) aufweist, der ein bevorzugtes Gebiet des Ablösens der Verschlussfolie (5) von dem Gehäuse (3) definiert, um es zu ermöglichen, dass die volatile Substanz (2) von dem Hohlraum (4) zu der Kammer (11) gelangt, wenn der Luftauffrischer (1) durch eine Kraft (F) aktiviert wird, die auf das Gehäuse (3) drückt, **dadurch gekennzeichnet, dass** die Kammer (11) auf einer Seite von der atmungsfähigen Folie (8) und auf der gegenüberliegende Seite von der Verschlussfolie (5) und von der Wand (12) des Gehäuses (3) eingeschlossen ist.

2. Luftauffrischer (1) nach Anspruch 1, bei dem der geformte Bereich (10) eine Form wie ein "V" hat, mit einer Spitze, die zu der Mitte des Hohlraums (4) zeigt.

3. Luftauffrischer (1) nach Anspruch 1 oder 2, bei dem der erste Grenzbereich (6) eine kleinere Breite (L2) als der geformte Bereich (10) hat.

4. Luftauffrischer (1) nach irgendeinem vorhergehenden Anspruch, bei dem eine äußere Oberfläche des Gehäuses (3) eine Vertiefung (13) aufweist, die geeignet ist, den Finger eines Benutzers aufzunehmen.

5. Luftauffrischer (1) nach irgendeinem vorhergehenden Anspruch, bei dem die Kammer (11) ein Material aufweist, das mit der volatilen Substanz (2) mischbar ist, oder die volatile Substanz (2) aufnimmt, die aus einer Gruppe ausgewählt ist, die Folgendes aufweist: eine Flüssigkeit, ein Pulver, ein absorbierendes Material, ein poröses Material, ein mit einer weiteren Substanz imprägniertes Material.

6. Luftauffrischer (1) nach irgendeinem vorhergehenden Anspruch, der ferner einen weiteren Hohlraum aufweist, der ein zusätzliches volatiles Material enthält, wobei der weitere Hohlraum der Kammer (11) zugewandt ist und durch eine betreffende weitere Verschlussfolie verschlossen ist.

## Revendications

1. Désodorisant comprenant une coque thermoformée (3) définissant une cavité (4) contenant une substance volatile (2), un film de fermeture (5) fixé à ladite coque (3) dans une première zone limite (6) entourant ladite cavité (4) pour fermer une ouverture (7) de ladite cavité (4), un film respirant (8) fixé à ladite coque (3) dans une deuxième zone limite (9) entourant ladite première zone limite (6) de sorte qu'une chambre (11) soit définie entre ledit film de fermeture (5), une paroi (12) de ladite coque (3) et ledit film respirant (8), ladite paroi (12) étant intercalée entre ladite première zone limite (6) et ladite deuxième zone limite (9), ledit film de fermeture (5) étant pelable sur ladite première zone limite (6), et ladite première zone limite (6) comprenant une partie profilée (10) définissant une superficie préférentielle de détachement dudit film de fermeture (5) de ladite coque (3) pour permettre à ladite substance volatile (2) de passer de ladite cavité (4) à ladite chambre (11) quand ledit désodorisant (1) est activé par une force (F) appuyant sur ladite coque (3), **caractérisé en ce que** ladite chambre (11) est délimitée sur un côté par ledit film respirant (8) et sur le côté opposé par ledit film de fermeture (5) et par ladite paroi (12) de ladite coque (3).

2. Désodorisant (1) selon la revendication 1, dans lequel ladite partie profilée (10) a un profil en "V" avec une pointe dirigée vers le centre de ladite cavité (4).

3. Désodorisant (1) selon la revendication 1 ou 2, dans lequel ladite première zone limite (6) a une plus petite largeur (L2) sur ladite partie profilée (10).

4. Désodorisant (1) selon l'une quelconque des revendications précédentes, dans lequel une surface extérieure de ladite coque (3) comprend un creux (13) approprié pour recevoir le doigt d'un utilisateur.

5. Désodorisant (1) selon l'une quelconque des revendications précédentes, dans lequel ladite chambre (11) contient un matériau apte à être mélangé à ladite substance volatile (2) ou recevant ladite substance volatile (2), sélectionné dans un groupe comprenant : un liquide, une poudre, un matériau absorbant, un matériau poreux, un matériau imprégné d'une autre substance.

6. Désodorisant (1) selon l'une quelconque des revendications précédentes et comprenant par ailleurs une cavité supplémentaire contenant un matériau volatil supplémentaire, ladite cavité supplémentaire faisant face à ladite chambre (11) et étant fermée par un film de fermeture supplémentaire respectif.
